# EUROPÄISCHE PATENTANMELDUNG

(11) **EP 4 647 091 A1**
(43) Veröffentlichungstag der Anmeldung: **12.11.2025**
(21) Anmeldenummer: 25173963.7
(22) Anmeldetag: 02.05.2025
(51) Int. Cl.: A61M 1/14, A61M 1/16

(54) **AUFNAHMEKÖCHER, SYSTEM AUS AUFNAHMEKÖCHER UND AUFNAHMESTAB, UND MEDIZINISCHE VORRICHTUNG MIT DEM SYSTEM**

(30) Priorität: 10.05.2024 DE 102024113130
(71) Anmelder: B. Braun Avitum AG, 34212 Melsungen (DE)
(72) Erfinder: KNIERIM, Michael, 37242 Bad Sooden-Allendorf (DE)
(74) Vertreter: Winter, Brandl - Partnerschaft mbB

(57) **Zusammenfassung**

Die vorliegende Offenbarung betrifft einen Aufnahmeköcher (12) einer oder für eine medizinische Vorrichtung (2), vorzugsweise einer oder für eine Vorrichtung zur extrakorporalen Blutbehandlung, mit einem Aufnahmeraum (16) und einer Druckausgleichsleitung (30). Der Aufnahmeraum (16) erstreckt sich ausgehend von einer in einer Oberfläche (4) der medizinischen Vorrichtung ausgebildeten Einführöffnung (10) in einer ersten Erstreckungsrichtung (X) in die medizinische Vorrichtung. Die Druckausgleichsleitung (30) verbindet zumindest einen von der Einführöffnung (10) in der ersten Erstreckungsrichtung (X) beabstandeten ersten Abschnitt (26) des Aufnahmeraums (16) mit einer die medizinische Vorrichtung umgebenden Umgebung (50) druckausgleichend. Die vorliegende Offenbarung betrifft weiterhin ein System aus einem Aufnahmeköcher (12) und einen Ansaugstab (14) und eine medizinische Vorrichtung (2).

## Beschreibung

### Technisches Gebiet

Die vorliegende Offenbarung betrifft einen Aufnahmeköcher einer oder für eine medizinische Vorrichtung, ein System aus einem Aufnahmeköcher und einem Aufnahmestab, und eine medizinische Vorrichtung mit dem System.

Medizinische Vorrichtungen wie Vorrichtungen zur extrakorporalen Blutbehandlung, insbesondere Dialysemaschinen/ Dialysegeräte, finden weit verbreitetet Einsatz im medizinischen Alltag. Derartige medizinische Vorrichtungen benötigen einer Vielzahl an unterschiedlichen Flüssigkeiten, um ihre bestimmungsgemäße Funktion zu erfüllen.

Einige der Flüssigkeiten können in der medizinischen Vorrichtung aus Konzentrat angemischt werden. Beispielsweise können Flüssigkeiten und/ oder Ausgangsstoffe/ Grundstoffe/ Mischungsbestandteile, welche in der medizinischen Vorrichtung weiterverarbeitet bzw. vermischt werden, über ein Ansaugsystem in die medizinische Vorrichtung eingebracht werden.

Derartige Ansaugsysteme beinhalten einen Ansaugstab oder mehrere Ansaugstäbe, der/ die vorgesehen und ausgebildet ist/ sind, in einen externen Kanister, der in der Regel auf einem Sockel der medizinischen Vorrichtung positioniert ist, eingeführt zu werden, um beispielsweise Bicarbonat und/ oder Konzentrat aus dem Kanister zu entnehmen/ anzusaugen.

Wenn der Ansaugstab nicht verwendet wird, ist dieser sicher und hygienisch zu verstauen, um eine Beschädigung und eine Kontamination zu verhindern. Hierfür ist in der medizinischen Vorrichtung ein Aufnahmeköcher ausgebildet, den Ansaugstab aufzunehmen um diesen zu lagern. In dem Aufnahmeköcher ist häufig eine Spülvorrichtung ausgebildet, einen Spülvorgang für den in dem Aufnahmeköcher gelagerten Ansaugstab durchzuführen, um diesen nach einer Benutzung zu spülen und/ oder zu desinfizieren und/ oder zu reinigen.

Während des Spülvorgangs wird eine Reinigungsflüssigkeit/ Spülflüssigkeit durch den Aufnahmeköcher gefördert, um den Ansaugstab zu reinigen und etwaige Rückstände der durch den Ansaugstab geförderten Flüssigkeit zu entfernen.

Üblicherweise verbleiben Reste der Reinigungsflüssigkeit nach dem Spülvorgang in dem Aufnahmeköcher. Da der Aufnahmeköcher mit dem Ansaugstab im Wesentlichen dicht verschlossen ist, um eine Verunreinigung des Ansaugstabs zu vermeiden, verdunstet die verbleibende Reinigungsflüssigkeit auch nicht aus dem Aufnahmeköcher.

Wenn dann der Ansaugstab zur erneuten Benutzung zügig aus dem Aufnahmeköcher gezogen wird, können durch einen in dem Aufnahmeköcher durch den dichten Sitz zwischen Ansaugstab und Aufnahmeköcher entstehender Unterdruck die Reste der Reinigungsflüssigkeit mit aus dem Aufnahmeköcher herausgezogen werden. Dies führt zu einer Verschmutzung der medizinischen Vorrichtung, was einerseits hygienische Probleme verursacht und andererseits Unsicherheiten bei einem Patienten über einen Pflegezustand der medizinischen Vorrichtung hervorrufen kann, was im Rahmen einer medizinischen Behandlung möglichst zu vermeiden ist.

### Kurzbeschreibung der Offenbarung

Aufgabe der vorliegenden Offenbarung ist es daher, die Nachteile aus dem Stand der Technik zu beheben oder zumindest zu reduzieren. Konkret ist es Aufgabe der vorliegenden Offenbarung, eine Möglichkeit bereitzustellen, eine Verschmutzung einer medizinischen Vorrichtung durch Reinigungsflüssigkeit zu verhindern oder zumindest ein Verschmutzungsrisiko zu minimieren.

Diese Aufgabe wird gelöst durch einen Aufnahmeköcher nach dem unabhängigen Anspruch 1, durch ein System nach Anspruch 9 sowie durch eine medizinische Vorrichtung nach Anspruch 14. Vorteilhafte Weiterbildungen sind in den Unteransprüchen beansprucht und/ oder nachfolgend beschrieben.

Konkret wird die Aufgabe der vorliegenden Offenbarung gelöst durch einen Aufnahmeköcher einer oder für eine medizinische Vorrichtung, insbesondere einer oder für eine Vorrichtung zur extrakorporalen Blutbehandlung, mit einem Aufnahmeraum und einer Druckausgleichsleitung bzw. einer Druckausgleichsverbindung, insbesondere einer Druckausgleichsnut, wobei sich der Aufnahmeraum ausgehend von einer, vorzugsweise in einer Oberfläche der medizinischen Vorrichtung ausgebildeten, Einführöffnung in einer ersten Erstreckungsrichtung, vorzugsweise in die medizinische Vorrichtung, erstreckt. Die Druckausgleichsleitung verbindet zumindest einen von der Einführöffnung in der ersten Erstreckungsrichtung beabstandeten/ getrennten oder sich von dieser erstreckenden ersten Abschnitt des Aufnahmeraums mit einer, vorzugsweise die medizinische Vorrichtung umgebenden, Umgebung druckausgleichend/ isobar.

In anderen Worten wird die Aufgabe gelöst durch den Aufnahmeköcher, der in der medizinischen Vorrichtung ausgebildet ist oder an die medizinische Vorrichtung adaptierbar/ anbringbar/ montierbar ist. Bei der medizinischen Vorrichtung kann es sich bevorzugt um eine Vorrichtung für die extrakorporale Blutbehandlung, insbesondere ein Dialysegerät/ eine Dialysemaschine handeln. Der Aufnahmeköcher beinhaltet zumindest den Aufnahmeraum, der vorzugsweise vorgesehen und ausgebildet ist, einen Ansaugstab oder ähnliche Elemente, welche reversibel in dem Aufnahmeköcher und damit in oder an der medizinischen Vorrichtung aufgenommen werden sollen, aufzunehmen.

Nachfolgend wird zum besseren Verständnis exemplarisch immer von dem Ansaugstab gesprochen, wenn ein Element gemeint ist, welches von dem Aufnahmeköcher aufgenommen ist oder aufgenommen werden soll. Selbstverständlich können auch andere geeignete Elemente in den offenbarungsgemäßen Aufnahmeköcher eingesetzt werden.

Der Aufnahmeraum erstreckt sich ausgehend von der Einführöffnung in der ersten Erstreckungsrichtung. Anders ausgedrückt bildet die Einführöffnung ein erstes Ende, vorzugsweise eine erste Stirnseite, des Aufnahmeraums. Die Einführöffnung ist vorgesehen und ausgebildet, den Aufnahmeraum von außen zugänglich zu machen. Beispielsweise ist die Einführöffnung vorgesehen und ausgebildet, dass der Ansaugstab zumindest teilweise, vorzugsweise vollständig, in den Aufnahmeraum eingeführt werden kann.

Der Aufnahmeraum hat einen ersten Abschnitt, welcher von der Einführöffnung beabstandet ausgebildet ist. Der erste Abschnitt kann vorgesehen und ausgebildet sein, einen Zentrierabschnitt des Ansaugstabs aufzunehmen und/ oder, vorzugsweise radial, insbesondere abschnittsweise radial umfänglich, zu kontaktieren.

Der erste Abschnitt ist bevorzugt zwischen der Einführöffnung und einem zweiten Abschnitt des Aufnahmeraums ausgebildet, wobei der zweite Abschnitt ein Abschnitt ist, in dem der Ansaugstab oder die ähnlichen Elemente mit der Reinigungsflüssigkeit gereinigt werden.

Der Aufnahmeköcher beinhaltet weiterhin die zumindest eine Druckausgleichsleitung. Die Druckausgleichsleitung verbindet den ersten Abschnitt mit der den Aufnahmeköcher umgebenden Umgebung derart, dass (Umgebungs-) Luft ungehindert durch die Druckausgleichsleitung strömen kann. Konkret kann die Luft bei einem Einsteckvorgang, also einem Vorgang, bei dem der Ansaugstab in den Aufnahmeköcher eingesetzt/ eingeschoben wird, durch die Druckausgleichsleitung aus dem Aufnahmeraum in die Umgebung entweichen. Weiterhin kann die Luft bei einem Ausziehvorgang, also einem Vorgang, bei dem der Ansaugstab aus dem Aufnahmeköcher herausgezogen/ entnommen wird, durch die Druckausgleichsleitung aus der Umgebung in den Aufnahmeraum strömen.

Durch eine derartige Ausbildung des Aufnahmeköchers kann ein Unterdruck in dem Aufnahmeraum bei dem Herausziehen des Ansaugstabs aus dem Aufnahmeköcher verhindert oder direkt abgebaut/ ausgeglichen werden. Konkret kann ein gleichmäßiger Druckausgleich zwischen der Umgebung und dem Aufnahmeraum beim Herausziehen des Ansaugstabs aus dem Aufnahmeköcher über die Druckausgleichsleitung erzielt werden, sodass ein Herausziehen/ Herausschleudern/ Heraussaugen von Reinigungsflüssigkeit aus dem Aufnahmeköcher verhindert werden kann. Entsprechend kann ein Überdruck in dem Aufnahmeraum bei dem Einführen des Ansaugstabs in den Aufnahmeköcher verhindert werden. Konkret kann der gleichmäßige Druckausgleich zwischen der Umgebung und dem Aufnahmeraum beim Einführen des Ansaugstabs in den Aufnahmeköcher über die Druckausgleichsleitung erzielt werden, sodass ein Herausdrücken von Reinigungsflüssigkeit aus dem Aufnahmeköcher verhindert werden kann. Auf diese Weise kann durch den über die Druckausgleichsleitung sichergestellten Druckausgleich eine Verschmutzung der medizinischen Vorrichtung effektiv verhindert werden.

In einem Aspekt kann der Aufnahmeraum zumindest abschnittsweise zylindrisch, vorzugsweise kreiszylindrisch, ausgebildet sein.

In anderen Worten kann der Aufnahmeraum in einer Draufsicht in der Erstreckungsrichtung eine zumindest abschnittsweise runde Querschnittsfläche aufweisen. Die Querschnittsfläche ist hierbei vorzugsweise kreisrund. Es sind jedoch auch Ausführungsformen vorstellbar, in denen die Querschnittsfläche eine ovale oder eine eckige, insbesondere eine vieleckige Geometrie aufweist.

Weiterhin kann der Aufnahmeraum abschnittsweise zylindrisch und abschnittsweise konisch/ kegelförmig/ kegelstumpfförmig ausgebildet sein. Alternativ oder zusätzlich kann der Aufnahmeraum gestuft ausgebildet sein. **In** anderen Worten kann die Querschnittsfläche über die Erstreckungsrichtung sprunghaft zu- oder abnehmen.

Durch eine derartige Ausbildung des Aufnahmeraums kann eine zuverlässige definierte (abschnittsweise) Abdichtung des Aufnahmeköchers und/ oder eine Zentrierung in dem Aufnahmeköcher erzielt werden.

In einem weiteren Aspekt kann der Aufnahmeraum durch eine innere Mantelfläche begrenzt sein und die Druckausgleichsleitung kann nutförmig parallel zu oder in der ersten Erstreckungsrichtung in der Mantelfläche ausgebildet sein.

In anderen Worten kann der Aufnahmeraum eine im Wesentlichen rohrförmige Mantelfläche aufweisen, wobei die Druckausgleichsleitung als eine Ausnehmung bzw. als ein Rücksprung in der Mantelfläche des Aufnahmeraums ausgebildet sein kann. **In** nochmals anderen Worten kann die Druckausgleichsleitung als die radial nach außen ausgebildete Ausnehmung bzw. als der Rücksprung in einer Richtung radial nach außen in der Mantelfläche des Aufnahmeraums ausgebildet sein und sich im Wesentlichen linear/ geradlinig parallel zu oder in der ersten Erstreckungsrichtung erstrecken.

Eine derartige Druckausgleichsleitung in der Mantelfläche des Aufnahmeraums kann kostengünstig und einfach ausgebildet/ hergestellt werden. Zusätzlich kann durch die hin zu dem Aufnahmeraum offene Ausbildung ein gleichmäßiger Druckausgleich/ eine gleichmäßige Luftströmung zwischen dem Aufnahmeraum und der Umgebung erzielt werden. Des Weiteren ist durch die zum Aufnahmeraum hin offene Gestaltung der Druckausgleichsleitung eine einfache Reinigung der Druckausgleichsleitung möglich.

Bevorzugt kann die Druckausgleichsleitung stoffeinstückig mit der Mantelfläche des Aufnahmeraums ausgebildet sein.

Es sind auch Ausführungsformen vorstellbar, in denen die Druckausgleichsleitung als eine Druckausgleichsbohrung in einem Winkel, beispielsweise einem 90° Winkel, zu dem Aufnahmeraum ausgebildet ist und lediglich punktuell/ punktförmig, insbesondere kreisförmig, in den Aufnahmeraum eingreift.

Weiterhin sind Ausführungsformen vorstellbar, in denen die Druckausgleichsleitung zwar parallel zu oder in der ersten Erstreckungsrichtung verläuft, jedoch nicht nutförmig hin zu dem Aufnahmeraum geöffnet ist, sondern lediglich zumindest eine aufnahmeraumseitige punktförmige Öffnung aufweist, welche die Mantelfläche perforiert. Es sind auch Ausführungsformen vorstellbar, in welchen zwischen der Druckausgleichsleitung und dem Aufnahmeraum eine Vielzahl an punktförmigen Öffnungen ausgebildet sind.

In einem weiteren Aspekt kann eine Nuttiefe der Druckausgleichsleitung in der radialen Richtung des Aufnahmeköchers/ des Aufnahmeraums über eine Längserstreckung der Druckausgleichsleitung in der Erstreckungsrichtung im Wesentlichen konstant sein. Unter im Wesentlichen konstant ist zu verstehen, dass die Nuttiefe bis auf eine etwaige geringfügige herstellprozessbedingte Änderung konstant ist.

In anderen Worten kann eine Druckausgleichsleitungsquerschnittsfläche über die Längserstreckung der Druckausgleichsleitung in der Erstreckungsrichtung im Wesentlichen konstant sein.

Die Druckausgleichsleitungsquerschnittsfläche kann eine teilkreisförmige Fläche sein oder beinhalten. Alternativ kann die Druckausgleichsleitungsquerschnittsfläche eine rechteckige Fläche sein oder beinhalten. Selbstverständlich sind auch weitere Geometrien der Druckausgleichsleitungsquerschnittsfläche vorstellbar.

In einer alternativen Ausführungsform kann sich die Nuttiefe über die Längserstreckung der Druckausgleichsleitung verändern. So kann sich die Nuttiefe hin zu der Einführöffnung vergrößern. In einer derartigen alternativen Ausführungsform kann erzielt werden, dass die Druckausgleichsleitung bei dem Ausziehvorgang des Ansaugstabs aus dem Aufnahmeköcher langsam geöffnet wird und so ein Luftströmungsvolumen kontinuierlich erhöht wird.

In einem weiteren Aspekt kann in der Druckausgleichsleitung ein Filter oder Gitterelement ausgebildet sein, das vorgesehen und ausgebildet ist, ein Eindringen von Fremdkörpern in den Aufnahmeraum über die Druckausgleichsleitung zu verhindern.

In einem weiteren Aspekt kann ein Übergang von der Druckausgleichsleitung zu der inneren Mantelfläche mit einem Radius ausgebildet sein.

Anders ausgedrückt können Nutkanten der insbesondere nutförmig ausgebildeten Druckausgleichsleitung mit Radien versehen sein/ abgerundet sein.

Durch eine Ausbildung der Druckausgleichsleitung mit Radien kann eine (schleichende) Beschädigung des Ansaugstabs und/ oder eines Dichtelements, beispielsweise einem O-Ring, des Ansaugstabsbeim Entlanggleiten an der Druckausgleichsleitung bei dem Einführ- und Ausziehvorgang verhindert werden und so eine Lebensdauer des Ansaugstabs und insbesondere des Dichtabschnitts des Ansaugstabs erheblich verlängert werden.

In einem weiteren Aspekt kann die Einführöffnung eine sich in der ersten Erstreckungsrichtung, vorzugsweise konisch, verjüngende Einführfase aufweisen.

In einem weiteren Aspekt kann ein erstes, zu der Umgebung hin offenes Ende der Druckausgleichsleitung in der Einführfase ausgebildet sein.

Anders ausgedrückt kann sich der Aufnahmeraum hin zu der Umgebung konisch aufweiten. Bevorzugt kann sich der Aufnahmeraum kegelstumpfförmig hin zu der Umgebung aufweiten.

In dem kegelstumpfförmigen Abschnitt kann das erste, zu der Umgebung hin offene Ende der Druckausgleichsleitung ausgebildet sein. In anderen Worten kann die Druckausgleichsleitung in dem konisch ausgebildeten Abschnitt der Einführöffnung ausgebildet sein.

Eine derartige Druckausgleichsleitung bzw. eine derartige Anordnung der Druckausgleichsleitung ist einfach und mit einfachen Werkzeugen, beispielsweise mit einem einfachen Spritzgießwerkzeug, herstellbar. Weiterhin ist eine derartige Druckausgleichsleitung einfach zu reinigen.

Eine Fasenbreite F der Einführfase kann bevorzugt größer sein als die Nuttiefe N der Druckausgleichsleitung. Insbesondere kann F ≥ √(2N²) sein.

In einem weiteren Aspekt kann die Druckausgleichsleitung an einer von einer Aufstandsfläche/ einem Sockel/ einer Kanisteraufnahme der medizinischen Vorrichtung abgewandten Seite des Aufnahmeraums ausgebildet sein.

In anderen Worten kann die Druckausgleichsleitung an einer Oberseite des Aufnahmeraums ausgebildet sein. In nochmals anderen Worten kann die Druckausgleichsleitung so in oder an oder zu dem Aufnahmeraum ausgebildet sein, dass die Druckausgleichsleitung nach unten, also in Schwerkraftrichtung, geöffnet ist.

Durch eine derartige Ausbildung des Aufnahmeköchers kann verhindert werden, dass (stehende) Flüssigkeit in der Druckausgleichsleitung verbleibt. Es kann sichergestellt sein, dass etwaige Reinigungsflüssigkeit oder Restflüssigkeit, welche durch den Ansaugstab gefördert wurde, aus der Druckausgleichsleitung zurück in den Aufnahmeraum läuft. Auf diese Weise kann effektiv verhindert werden, dass der Luftstrom durch die Druckausgleichsleitung Restflüssigkeit mit sich zieht und die medizinische Vorrichtung verschmutzt.

In einem weiteren Aspekt können mehrere Druckausgleichsleitungen, vorzugsweise gleichmäßig, über einen Umfang des Aufnahmeraums verteilt ausgebildet sein.

Anders ausgedrückt können mehrere, vorzugsweise zwei, drei, vier, fünf, sechs oder mehr Druckausgleichsleitungen, vorzugweise mit einem konstanten Winkelabstand, in einer Draufsicht in der ersten Erstreckungsrichtung zueinander über die Mantelfläche des Aufnahmeraums verteilt ausgebildet sein.

Durch eine Vielzahl an Druckausgleichsleitungen kann eine Luftstromgeschwindigkeit in den einzelnen Druckausgleichsleitungen reduziert werden. So kann verhindert werden, dass die Luft in der Druckausgleichsleitung düsenförmig beschleunigt wird und etwaige Tropfen von Restflüssigkeit von der Luftströmung mitgerissen werden.

Weiterhin wird die Aufgabe der vorliegenden Offenbarung gelöst durch ein System aus einem Aufnahmeköcher einer oder für eine medizinische Vorrichtung, insbesondere einer oder für eine Vorrichtung zur extrakorporalen Blutbehandlung, vorzugsweise einem Aufnahmeköcher wie voranstehend beschrieben, mit einem Aufnahmeraum, wobei sich der Aufnahmeraum ausgehend von einer (in einer Oberfläche ausgebildeten) Einführöffnung in einer ersten Erstreckungsrichtung (in die medizinische Vorrichtung) erstreckt, und einem Ansaugstab, wobei der Ansaugstab vorgesehen und ausgebildet ist, in den Aufnahmeköcher eingebracht zu werden und einen Dichtabschnitt aufweist, der vorgesehen und ausgebildet ist, radial umlaufend gegen den Aufnahmeköcher zu dichten. In dem Aufnahmeköcher und/ oder in dem Ansaugstab ist eine Druckausgleichsleitung ausgebildet, zumindest einen von der Einführöffnung in der ersten Erstreckungsrichtung beabstandeten oder sich von dieser erstreckenden ersten Abschnitt des Aufnahmeraums mit einer (die medizinische Vorrichtung umgebenden) Umgebung druckausgleichend/ isobar zu verbinden.

In anderen Worten wird die Aufgabe gelöst durch das System aus dem Aufnahmeköcher für eine medizinische Vorrichtung, vorzugsweise für ein Dialysegerät/ eine Dialysemaschine und dem Ansaugstab.

Der Aufnahmeköcher beinhaltet zumindest den Aufnahmeraum, der vorzugsweise vorgesehen und ausgebildet ist, den Ansaugstab oder ähnliche Elemente, welche reversibel in dem Aufnahmeköcher aufgenommen werden können oder sind, aufzunehmen.

Der Aufnahmeraum erstreckt sich ausgehend von der Einführöffnung in der ersten Erstreckungsrichtung, vorzugsweise zumindest abschnittsweise zylindrisch. Der Aufnahmeraum hat einen ersten Abschnitt, welcher von der Einführöffnung beabstandet ausgebildet ist. Der erste Abschnitt kann vorgesehen und ausgebildet sein, einen Zentrierabschnitt des Ansaugstabs aufzunehmen.

Der Ansaugstab ist vorgesehen und ausgebildet, in einen Kanister oder einen vergleichbaren Behälter eingebracht/ eingehängt/ eingesteckt zu werden und aus dem Kanister Flüssigkeit in die medizinische Vorrichtung zu fördern/ zu transportieren. Der Ansaugstab hat einen Dichtabschnitt, der in dem eingesetzten Zustand, also in dem Zustand in dem der Ansaugstab in dem Aufnahmeköcher untergebracht/ eingebracht/ eingesteckt ist, radial gegen den Aufnahmeköcher dichtet. Zumindest ein Dichtelement kann hierbei in/ an dem Ansaugstab und/ oder in/ an dem Aufnahmeköcher ausgebildet sein.

In dem Ansaugstab und/ oder in dem Aufnahmeköcher und/ oder zwischen Ansaugstab und Aufnahmeköcher ist die zumindest eine Druckausgleichsleitung ausgebildet, den ersten Abschnitt mit der Umgebung derart zu verbinden, dass (Umgebung-) Luft ungehindert durch die Druckausgleichsleitung strömen kann. Gemäß der vorliegenden Offenbarung kann somit alternativ oder zusätzlich die Druckausgleichsleitung, beispielsweise in Form einer Nut, auch in dem Ansaugstab ausgebildet sein. Konkret kann die Luft bei einem Einsteckvorgang, also einem Vorgang, bei dem der Ansaugstab in den Aufnahmeköcher eingesetzt wird, durch die Druckausgleichsleitung aus dem Aufnahmeraum in die Umgebung entweichen. Weiterhin kann die Luft bei einem Ausziehvorgang, also einem Vorgang, bei dem der Ansaugstab aus dem Aufnahmeköcher herausgezogen wird, durch die Druckausgleichsleitung aus der Umgebung in den Aufnahmeraum strömen.

Durch eine derartige Ausbildung des Aufnahmeköchers kann ein Unterdruck in dem Aufnahmeraum bei dem Herausziehen des Ansaugstabs aus dem Aufnahmeköcher verhindert werden. Konkret kann ein gleichmäßiger Druckausgleich zwischen der Umgebung und dem Aufnahmeraum beim Herausziehen des Ansaugstabs aus dem Aufnahmeköcher über die Druckausgleichsleitung erzielt werden, sodass ein Herausziehen von Reinigungsflüssigkeit aus dem Aufnahmeköcher verhindert werden kann.

Entsprechend kann ein Überdruck in dem Aufnahmeraum bei dem Einführen/ Einstecken des Ansaugstabs in den Aufnahmeköcher verhindert werden. Konkret kann der gleichmäßige Druckausgleich zwischen der Umgebung und dem Aufnahmeraum beim Einführen des Ansaugstabs in den Aufnahmeköcher über die Druckausgleichsleitung erzielt werden, sodass ein Herausdrücken von Reinigungsflüssigkeit aus dem Aufnahmeköcher verhindert werden kann. Auf diese Weise kann eine Verschmutzung der medizinischen Vorrichtung effektiv verhindert werden.

In einem Aspekt kann in dem eingesetzten Zustand des Ansaugstabs in dem Aufnahmeköcher der Dichtabschnitt in der ersten Erstreckungsrichtung hinter dem ersten Abschnitt ausgebildet sein und den zweiten Abschnitt des Aufnahmeraums gegen die Umgebung abdichten.

In anderen Worten ist der erste Abschnitt bevorzugt zwischen der Einführöffnung und dem zweiten Abschnitt des Aufnahmeraums ausgebildet, wobei der zweite Abschnitt ein Abschnitt ist, in dem der Ansaugstab oder die ähnlichen Elemente in dem eingesetzten Zustand mit der Reinigungsflüssigkeit/ Spülflüssigkeit gereinigt/ gespült werden.

In dem eingesetzten Zustand kann der Dichtabschnitt vorzugsweise zwischen dem ersten Abschnitt und dem zweiten Abschnitt ausgebildet sein. In anderen Worten kann der Dichtabschnitt den ersten Abschnitt von dem zweiten Abschnitt trennen.

In einem weiteren Aspekt kann der Ansaugstab einen Zentrierabschnitt aufweisen/ beinhalten, welcher in dem eingesetzten Zustand den Ansaugstab, vorzugsweise in Verbindung mit dem Dichtabschnitt, in dem Aufnahmeköcher zentriert.

In anderen Worten kann der Ansaugstab den Zentrierabschnitt beinhalten, der vorgesehen und ausgebildet ist, den Ansaugstab in dem Aufnahmeköcher radial zu zentrieren. Der Zentrierabschnitt kann in dem eingesetzten Zustand näher an der Einführöffnung positioniert sein als der Dichtabschnitt.

Durch einen derartigen Zentrierabschnitt kann sichergestellt sein, dass der Dichtabschnitt in vorbestimmter Weise orientiert/ angeordnet ist und somit eine zuverlässige Abdichtung gewährleistet ist.

Der Zentrierabschnitt kann weiterhin als eine Fremdkörpersperre fungieren und verhindern, dass Fremdkörper in den Aufnahmeköcher eindringen können.

In einem weiteren Aspekt kann der Aufnahmeköcher oder der Ansaugstab in dem Dichtabschnitt und/ oder in dem Zentrierabschnitt zumindest einen O-Ring beinhalten.

In anderen Worten kann der Ansaugstab einen umfänglich angeordneten O-Ring in dem Dichtabschnitt und/ oder in dem Zentrierabschnitt beinhalten.

Alternativ oder zusätzlich kann der Aufnahmeköcher in der Mantelfläche des Aufnahmeköchers an einer Position, in welcher in dem eingesetzten Zustand der Dichtabschnitt und/ oder der Zentrierabschnitt des Ansaugstabs positioniert ist, zumindest einen O-Ring beinhalten.

In dem Dichtabschnitt kann der O-Ring als Dichtelement fungieren. Es sind auch andere Dichtelemente, wie beispielsweise eine Radialdichtung, beipsielsweise in Form eines Quadrings, oder einer Axialdichtung, vorstellbar.

In dem Zentrierabschnitt kann der O-Ring als ein (klemmendes) Zentrierelement fungieren. Es sind auch andere Zentrierelemente, wie beispielsweise ein auf dem Ansaugstab ausgebildeter Wellenabsatz, vorstellbar.

In einem weiteren Aspekt kann zwischen dem Aufnahmeraum und dem Ansaugstab in einem eingesetzten Zustand zumindest abschnittsweise eine Presspassung ausgebildet sein.

In anderen Worten kann in dem eingesetzten Zustand, vorzugsweise in dem Dichtabschnitt und/ oder in dem Zentrierabschnitt eine Presspassung oder ein Klemmsitz zwischen der Mantelfläche des Aufnahmeköchers und dem Ansaugstab ausgebildet sein.

Alternativ oder zusätzlich kann eine punktuelle Verjüngung des Durchmessers des Aufnahmeköchers, beispielsweise in Form eines, vorzugsweise punktuellen, Vorsprungs, ausgebildet sein, wobei der Vorsprung einen Widerstand für das Herausziehen des O-Rings ausbildet.

Eine derartige Presspassung fixiert den Ansaugstab in dem Aufnahmeköcher und verhindert ein unbeabsichtigtes Herausziehen oder Herausrutschen des Ansaugstabs aus dem Aufnahmeköcher. Über die Art der Presspassung und vorzugsweise über eine Stärke des O-Rings kann eine Klemmkraft zwischen dem Ansaugstab und dem Aufnahmeköcher einstellbar sein.

Weiterhin wird die Aufgabe der vorliegenden Offenbarung gelöst durch eine medizinische Vorrichtung, vorzugsweise eine Vorrichtung zur extrakorporalen Blutbehandlung, insbesondere ein Dialysegerät/ eine Dialysemaschine, mit einem System nach einem der obigen Aspekte.

Konkret wird die Aufgabe gelöst durch die medizinische Vorrichtung, welche das offenbarungsgemäße System aus dem Aufnahmeköcher und dem Ansaugstab beinhaltet. Das System kann in die medizinische Vorrichtung integriert oder an die medizinische Vorrichtung adaptiert/ montiert sein.

Vorzugsweise kann die medizinische Vorrichtung mehr als ein offenbarungsgemäßes System, insbesondere zwei, drei, vier oder mehr offenbarungsgemäße Systeme beinhalten.

In einem Aspekt kann der Ansaugstab ein Ansaugstab zum Ansaugen von Bicarbonat und/ oder Konzentrat aus einem externen Behälter sein und der Aufnahmeköcher kann vorgesehen und ausgebildet sein, den Ansaugstab zu lagern und zu spülen.

### Kurzbeschreibung der Figuren

Fig. 1 ist eine perspektivische Teildarstellung einer offenbarungsgemäßen medizinischen Vorrichtung in Form eines Dialysegeräts mit zwei offenbarungsgemäßen Aufnahmeköchern;
Fig. 2 ist eine erste Schnittansicht durch den offenbarungsgemäßen Aufnahmeköcher mit einem Ansaugstab, wobei der Ansaugstab vollständig in den Aufnahmeköcher eingesetzt ist;
Fig. 3 ist eine zweite Schnittansicht durch den offenbarungsgemäßen Aufnahmeköcher mit dem Ansaugstab, wobei der Ansaugstab ein erstes Stück aus dem Aufnahmeköcher ausgezogen ist;
Fig. 4 ist eine dritte Schnittansicht durch den offenbarungsgemäßen Aufnahmeköcher mit dem Ansaugstab, wobei der Ansaugstab ein zweites Stück, welches größer ist als das erste Stück, aus dem Aufnahmeköcher ausgezogen ist; und
Fig. 5 zeigt eine vierte Schnittansicht durch den offenbarungsgemäßen Aufnahmeköcher, wenn der Ansaugstab vollständig aus dem Aufnahmeköcher ausgezogen ist.

### Beschreibung der Ausführungsbeispiele

Nachstehend werden Ausführungsbeispiele der vorliegenden Offenbarung auf der Basis der zugehörigen Figuren beschrieben.

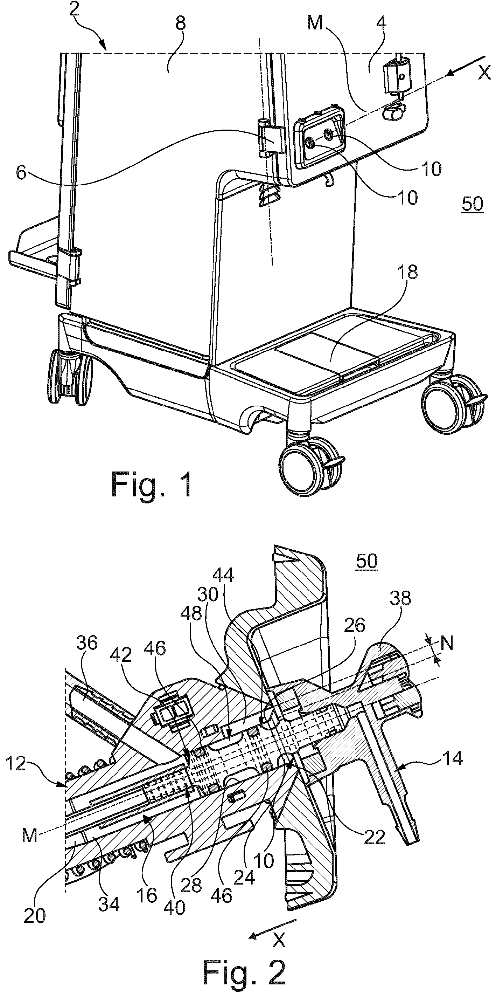

Fig. 1 zeigt eine medizinische Vorrichtung in Form eines Dialysegeräts 2. Das Dialysegerät 2 hat eine Frontplatte 4, welche über ein Scharnier 6 an einem Gehäuse 8 des Dialysegeräts 2 anscharniert ist. **In** die Frontplatte 4 des Dialysegeräts 2 sind regelmäßig verschiedenste Aggregate und Steckplätze integriert, wie beispielsweise Peristaltikpumpen, Infusoren und dergleichen.

Weiterhin sind in der Frontplatte 4 Einführöffnungen 10 zu Aufnahmeköchern 12 (siehe Fign. 2 bis 5) ausgebildet. Durch die Einfuhröffnungen 10 können Ansaugstäbe 14 in Aufnahmeräume 16 der Aufnahmeköcher 12 gesteckt/ eingebracht werden, um die Ansaugstäbe 14 zu lagern und zu spülen. Die Ansaugstäbe 14 sind über Schläuche oder sonstige Leitungen (nicht dargestellt) an das Dialysegerät 2 angeschlossen und sind vorgesehen und ausgebildet, in Kanister (nicht dargestellt) eingebracht zu werden, welche beispielsweise auf einer Bodenplatte/ einem Sockel 18 des Dialysegeräts 2 unterhalb der Frontplatte 4 positioniert sind. Die Ansaugstäbe 14 sind vorgesehen und ausgebildet, Flüssigkeiten, wie beispielsweise Bicarbonat und/ oder Konzentrat aus dem Kanister in das Dialysegerät 2 zu fördern. Die Aufnahmeköcher 12 sind im gezeigten Ausführungsbeispiel identisch ausgebildet. Es sind jedoch auch Ausführungsformen vorstellbar, in denen die Aufnahmeköcher 12 unterschiedlich dimensioniert ausgebildet sind.

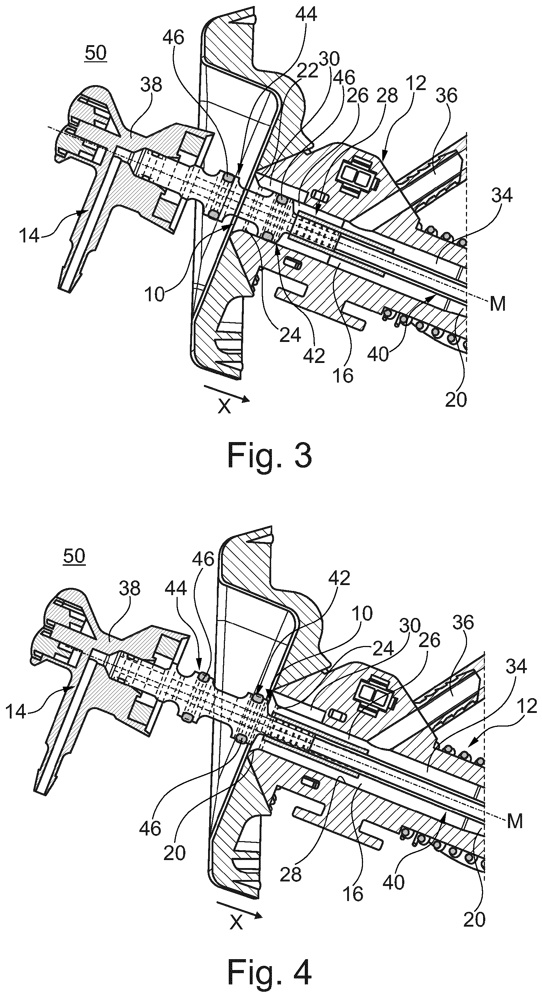

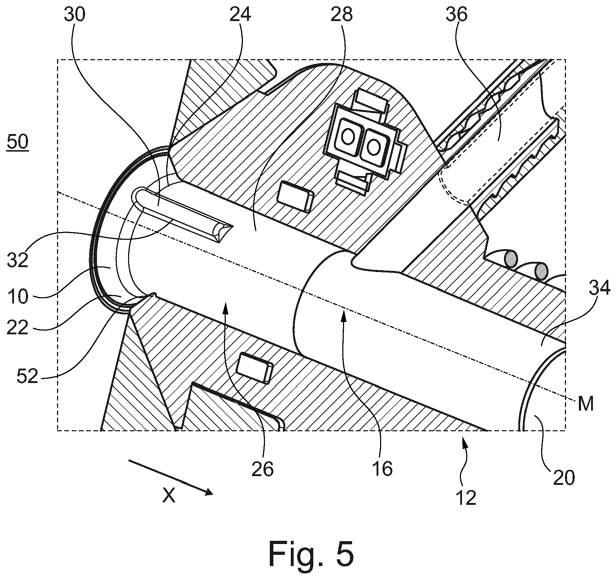

Fign. 2 bis 5 zeigen den offenbarungsgemäßen Aufnahmeköcher 12 mit dem Ansaugstab 14 in einer Schnittdarstellung, wobei der Ansaugstab 14 in Fig. 2 vollständig in den Aufnahmeköcher 12 eingeschoben ist und über Fig. 3 und Fig. 4 immer weiter aus dem Aufnahmeköcher 12 ausgezogen wird, bis der Ansaugstab 14 in Fig. 5 nicht mehr in dem Aufnahmeköcher 12 aufgenommen, sondern der Aufnahmeköcher 12 leer dargestellt ist.

Der Aufnahmeköcher 12 beinhaltet den Aufnahmeraum 16, welcher sich von der Einführöffnung 10 in einer ersten Erstreckungsrichtung X weg erstreckt. Anders ausgedrückt erstreckt sich der Aufnahmeraum 16 ausgehend von der Frontplatte 4 des Dialysegerät 2 in das Gehäuse 8 des Dialysegerät 2. Der Aufnahmeraum 16 ist abschnittsweise zylindrisch, in der hier dargestellten Ausführungsform kreiszylindrisch ausgebildet, um sich an einem von der Einführöffnung 10 abgewandten Endabschnitt 20 in der ersten Erstreckungsrichtung X konisch zu verjüngen.

Die Einführöffnung 10 ist mit einer Einführfase 22 ausgebildet. Anders ausgedrückt verjüngt sich die Einführöffnung 10 in der ersten Erstreckungsrichtung X konisch. An die Einführfase 22 schließt sich in der ersten Erstreckungsrichtung X ein Übergangsradius 24 an. An den Übergangsradius 24 schließt sich in der ersten Erstreckungsrichtung X ein erster Abschnitt 26 des Aufnahmeraums 16 an. Der erste Abschnitt 26 hat eine kreiszylindrische Geometrie.

In einer Mantelfläche 28 des ersten Abschnitts 26 ist eine Druckausgleichsleitung in Form einer Nut 30 ausgebildet. Die Nut 30 erstreckt sich mit einer konstanten Nuttiefe N in der Erstreckungsrichtung X in der Mantelfläche 28 des ersten Abschnitts 26. Die Nuttiefe N ist eine Erstreckung der Nut 30 in radialer Richtung bezogen auf eine Mittelfaser M des Aufnahmeköchers 12. Eine Erstreckung der Nut 30 in der ersten Erstreckungsrichtung X entspricht im Wesentlichen einer halben Erstreckung des ersten Abschnitt 26 in der ersten Erstreckungsrichtung X. Die Nut 30 mündet in der Einführfase 22 des Aufnahmeköchers 12 und hat in einer Draufsicht in der ersten Erstreckungsrichtung X eine teilkreisförmige Querschnittsfläche.

An einem Übergang von der Nut 30 in die Mantelfläche 28 ist ein Nutradius 32 ausgebildet. Anders ausgedrückt ist eine Kante zwischen Nut 30 und Mantelfläche 28 verrundet.

Die Nut 30 ist in der hier dargestellten Ausführungsform über eine vollständige Erstreckung der Nut 30 in der ersten Erstreckungsrichtung X offen hin zu dem Aufnahmeraum 16 ausgebildet. Es sind auch Ausführungsformen vorstellbar, in welchen die Druckausgleichsleitung nicht in Form der Nut 30, sondern in Form einer (Durchgangs-) Bohrung mit zumindest zwei Endöffnungen oder dergleichen ausgebildet ist.

In der ersten Erstreckungsrichtung X hinter dem ersten Abschnitt 26 ist ein zweiter Abschnitt 34 ausgebildet. Der zweite Abschnitt 34 hat eine (geringfügig) konische Geometrie. Der zweite Abschnitt 34 kann in einer alternativen Ausführungsform im Wesentlichen eine zylindrische Geometrie, insbesondere eine kreiszylindrische Geometrie aufweisen. In dem zweiten Abschnitt 34 ist in der Mantelfläche 28 ein Auslass einer Spülleitung 36 ausgebildet. Die Spülleitung 36 ist vorgesehen und ausgebildet, Spülflüssigkeit in den zweiten Abschnitt 34 des Aufnahmeraums 16 einzuleiten, um den Ansaugstab 14 zu reinigen und zu spülen.

Der Ansaugstab 14 hat einen Griffabschnitt 38, der vorgesehen und ausgebildet ist, von einem Bediener/ Behandler gegriffen zu werden und einen Stababschnitt 40, der vorgesehen und ausgebildet ist, in den Kanister eingeführt/ eingebracht zu werden. Der Stababschnitt 40 wird zur Reinigung und Aufbewahrung vollständig in den Aufnahmeköcher 12 eingeführt. In anderen Worten schlägt der Griffabschnitt 38 an der Frontplatte 4 an, wenn der Ansaugstab 14 vollständig in den Aufnahmeköcher 12 eingesetzt ist.

An dem Stababschnitt 40 ist ein Dichtabschnitt 42 und ein Zentrierabschnitt 44 ausgebildet. In der hier dargestellten Ausführungsform sind der Dichtabschnitt 42 und der Zentrierabschnitt 44 im Wesentlichen identisch, also auch mit einem identischen Durchmesser, ausgebildet. Selbstverständlich sind auch Ausführungsformen vorstellbar, in welchen der Dichtabschnitt 42 und der Zentrierabschnitt 44 unterschiedlich ausgebildet sind. Weiterhin sind Ausführungsformen vorstellbar, in welchen mehr als ein Dichtabschnitt 42 und/ oder mehr als ein Zentrierabschnitt 44 an dem Stababschnitt 40 ausgebildet sind.

Sowohl der Dichtabschnitt 42 als auch der Zentrierabschnitt 44 sind in der hier dargestellten Ausführungsform mit einem umlaufenden O-Ring 46 ausgebildet. Der O-Ring 46 des Dichtabschnitts 42 ist vorgesehen und ausgebildet, den zweiten Abschnitt 34 des Aufnahmeraums 16 hin zu der Einführöffnung 10 abzudichten, sodass in dem zweiten Abschnitt 34, vorzugsweise mit erhöhtem Druck, gespült und gereinigt werden kann, ohne dass die Spülflüssigkeit aus der Einführöffnung 10 austritt. Der Dichtabschnitt 42 trennt im Wesentlichen den ersten Abschnitt 26 von dem zweiten Abschnitt 34.

Der Zentrierabschnitt 44 ist vorgesehen und ausgebildet, den Ansaugstab 14 in dem ersten Abschnitt 26 zu zentrieren. Der Zentrierabschnitt 44 kann zusätzlich eine geringfügig dichtende Wirkung haben, es sei jedoch explizit darauf hingewiesen, dass die Nut 30 den Zentrierabschnitt 44 in dem eingesetzten Zustand überbrückt, sodass eine vollständige Abdichtung durch den Zentrierabschnitt 44 gerade nicht ausgebildet ist.

An der Einführöffnung 10 ist in einer alternativen Ausführungsform, wie in Fig. 5 dargestellt, ein (punktueller) Vorsprung 52 ausgebildet, welcher die Querschnittsfläche des Aufnahmeköchers 12 lokal (punktuell) reduziert. Der Vorsprung 52 bildet einen Widerstand, welcher von dem O-Ring 46 beim Herausziehen des Ansaugstabs 14 aus dem Aufnahmeköcher 12 überwunden werden muss.

Nachfolgend wird ein Ausziehvorgang des Ansaugstabs 14 aus dem Aufnahmeköcher 12 anhand der Fign. 2 bis 5 beschrieben.

In dem vollständig eingesetzten Zustand in Fig. 2 dichtet der Dichtabschnitt 42 des Ansaugstabs 14 gegen die Mantelfläche 28 des Aufnahmeraums 16 ab und segmentiert/ unterteilt den Aufnahmeraum 16 in den zweiten Abschnitt 34, in welchen über die Spülleitung 36 die Spülflüssigkeit eingebracht werden kann, um den Ansaugstab 14 zu spülen und zu reinigen, und den ersten Abschnitt 26. In dem ersten Abschnitt 26 ist in dem eingesetzten Zustand der Zentrierabschnitt 44 angeordnet. Der Zentrierabschnitt 44 kontaktiert radial umfänglich (bis auf den Bereich der Nut 30) die Mantelfläche 28 des Aufnahmeraums 16.

Ein Zwischenbereich 48 zwischen dem Zentrierabschnitt 44 und dem Dichtabschnitt 42 ist über die Nut 30 mit einer Umgebung 50 verbunden.

In Fig. 3 ist der Ansaugstab 14 um einen ersten Betrag aus dem Aufnahmeköcher 12 ausgezogen. In anderen Worten ist der Ansaugstab 14 in einer Richtung, die entgegen der ersten Erstreckungsrichtung X orientiert ist, aus dem Aufnahmeköcher 12 soweit ausgezogen, dass der Dichtabschnitt 42 in dem ersten Abschnitt 26 angeordnet ist. Anders ausgedrückt überbrückt in diesem Zustand die Nut 30 den Dichtabschnitt 42, sodass Umgebungsluft aus der Umgebung 50 durch die Nut 30 in den Aufnahmeraum 16 und insbesondere in den zweiten Abschnitt 34 des Aufnahmeraums 16 strömen kann. So kann ein Unterdruck, der durch das Ausziehen des Ansaugstabs 14 in dem zweiten Abschnitt 34 des Aufnahmeraums 16 entsteht, über die Nut 30 abgebaut werden, sodass effektiv verhindert werden kann, dass Flüssigkeit in dem in Fig. 4 dargestellten ausgezogenen Zustand aus dem Aufnahmeraum 16 geschleudert/ gespritzt wird und das Dialysegerät 2 verschmutzt.

Auf gleiche Weise wird über die Nut 30 bei einem Einführvorgang/ Einsetzvorgang des Ansaugstabs 14 in den Aufnahmeköcher 12 ein Überdruck in dem Aufnahmeraum 16 und insbesondere in dem zweiten Abschnitt 34 des Aufnahmeraums 16 abgebaut, was einerseits ein Herausdrücken der Flüssigkeit aus dem Aufnahmeköcher 12 verhindert und andererseits einen Einführkomfort für den Bediener erhöht.

Zusammengefasst fungiert die Nut 30 als Druckausgleichselement/ Druckausgleichsleitung, welche bei dem Einführvorgang und Ausziehvorgang des Ansaugstabs 14 in den Aufnahmeköcher 12 für einen Druckausgleich vor und hinter dem Dichtabschnitt 42 sorgt.

### Bezugszeichenliste

- 2: Dialysegerät/ medizinische Vorrichtung/ Vorrichtung zur extrakorporalen Blutbehandlung
- 4: Frontplatte/ Oberfläche
- 6: Scharnier
- 8: Gehäuse
- 10: Einführöffnung
- 12: Aufnahmeköcher
- 14: Ansaugstab
- 16: Aufnahmeraum
- 18: Sockel
- 20: Endabschnitt
- 22: Einführfase
- 24: Übergangsradius
- 26: erste Abschnitt
- 28: Mantelfläche
- 30: Nut/ Druckausgleichsleitung/ Druckausgleichsverbindung
- 32: Nutradius
- 34: zweiter Abschnitt
- 36: Spülleitung
- 38: Griffabschnitt
- 40: Stababschnitt
- 42: Dichtabschnitt
- 44: Zentrierabschnitt
- 46: O-Ring
- 48: Zwischenbereich
- 50: Umgebung
- 52: Vorsprung
- X: erste Erstreckungsrichtung
- N: Nuttiefe

## Patentansprüche

1. Aufnahmeköcher (12) einer medizinischen Vorrichtung (2) oder zum Anbringen an der medizinischen Vorrichtung (2), vorzugsweise einer oder für eine Vorrichtung zur extrakorporalen Blutbehandlung, mit
einem Aufnahmeraum (16) und
einer Druckausgleichsleitung bzw. Druckausgleichsverbindung (30),
wobei sich der Aufnahmeraum (16) ausgehend von einer Einführöffnung (10) in einer ersten Erstreckungsrichtung (X) erstreckt und
wobei die Druckausgleichsleitung (30) zumindest einen von der Einführöffnung (10) in der ersten Erstreckungsrichtung (X) beabstandeten oder sich von dieser erstreckenden ersten Abschnitt (26) des Aufnahmeraums (16) mit einer Umgebung (50) druckausgleichend verbindet.

2. Aufnahmeköcher (12) nach Anspruch 1, wobei der Aufnahmeraum (16) zumindest abschnittsweise zylindrisch, vorzugsweise kreiszylindrisch ausgebildet ist.

3. Aufnahmeköcher (12) nach Anspruch 1 oder 2, wobei der Aufnahmeraum (16) durch eine innere Mantelfläche (28) begrenzt ist und sich die Druckausgleichsleitung (30) nutförmig parallel zu oder in der ersten Erstreckungsrichtung (X) in der Mantelfläche (28) erstreckt.

4. Aufnahmeköcher (12) nach Anspruch 3, wobei eine Nuttiefe (N) der Druckausgleichsleitung (30) in einer radialen Richtung des Aufnahmeköchers (12) über eine Längserstreckung der Druckausgleichsleitung (30) in der ersten Erstreckungsrichtung (X) im Wesentlichen konstant ist.

5. Aufnahmeköcher (12) nach Anspruch 3 oder 4, wobei ein Übergang von der Druckausgleichsleitung (30) zu der inneren Mantelfläche (28) mit einem Radius (32) ausgebildet ist.

6. Aufnahmeköcher (12) nach einem der Ansprüche 1 bis 5, wobei die Einführöffnung (10) eine sich in der ersten Erstreckungsrichtung (X), vorzugsweise konisch, verjüngende Einführfase (22) aufweist und ein erstes, zu der Umgebung (50) hin offenes Ende der Druckausgleichsleitung (30) in der Einführfase (22) ausgebildet ist.

7. Aufnahmeköcher (12) nach einem der Ansprüche 1 bis 6, wobei die Druckausgleichsleitung (30) an einer von einem Sockel (18) der medizinischen Vorrichtung (2) abgewandten Seite des Aufnahmeraums (16) ausgebildet ist.

8. Aufnahmeköcher (12) nach einem der Ansprüche 1 bis 7, wobei mehrere Druckausgleichsleitungen (30), vorzugsweise gleichmäßig, über einen Umfang des Aufnahmeraums (16) verteilt ausgebildet sind.

9. System aus
einem Aufnahmeköcher (12) einer oder für eine medizinische Vorrichtung (2), insbesondere einer oder für eine Vorrichtung zur extrakorporalen Blutbehandlung, mit einem Aufnahmeraum (16), wobei sich der Aufnahmeraum (16) ausgehend von einer Einführöffnung (10) in einer ersten Erstreckungsrichtung (X) erstreckt, und
einem Ansaugstab (14), wobei der Ansaugstab (14) vorgesehen und ausgebildet ist, in den Aufnahmeköcher (12) eingebracht zu werden und einen Dichtabschnitt (42) aufweist, der vorgesehen und ausgebildet ist, radial umlaufend gegen den Aufnahmeköcher (12) zu dichten,
wobei in dem Aufnahmeköcher (12) und/ oder in dem Ansaugstab (14) eine Druckausgleichsleitung (30) ausgebildet ist, zumindest einen von der Einführöffnung (10) in der ersten Erstreckungsrichtung (X) beabstandeten oder sich von dieser erstreckenden ersten Abschnitt (26) des Aufnahmeraums (16) mit einer Umgebung (50) druckausgleichend zu verbinden.

10. System nach Anspruch 9, wobei in einem eingesetzten Zustand des Ansaugstabs (14) in dem Aufnahmeköcher (12) der Dichtabschnitt (42) in der ersten Erstreckungsrichtung (X) hinter dem ersten Abschnitt (26) ausgebildet ist und einen zweiten Abschnitt (34) des Aufnahmeraums (16) gegen die Umgebung (50) abdichtet.

11. System nach Anspruch 10, wobei der Ansaugstab (14) einen Zentrierabschnitt (44) aufweist, welcher in dem eingesetzten Zustand in dem ersten Abschnitt (26) angeordnet ist und den Ansaugstab (14), vorzugsweise in Verbindung mit dem Dichtabschnitt (42), in dem Aufnahmeköcher (12) zentriert.

12. System nach Anspruch 11 **dadurch gekennzeichnet, dass** der Aufnahmeköcher (12) oder der Ansaugstab (14) in dem Dichtabschnitt (42) und/ oder in dem Zentrierabschnitt (44) zumindest einen O-Ring (46) beinhalten.

13. System nach Anspruch 9 in Verbindung mit einem Aufnahmeköcher (12) nach einem der Ansprüche 1 bis 8, wobei zwischen dem Aufnahmeraum (16) und dem Ansaugstab (14) in einem eingesetzten Zustand des Ansaugstabs (14) zumindest abschnittsweise eine Presspassung ausgebildet ist.

14. Medizinische Vorrichtung (2), vorzugsweise eine Vorrichtung zur extrakorporalen Blutbehandlung, insbesondere ein Dialysegerät, mit einem System nach einem der Ansprüche 9 bis 13.

15. Medizinische Vorrichtung (2) nach Anspruch 14, wobei der Ansaugstab (14) ein Ansaugstab (14) zum Ansaugen von Bicarbonat und/ oder Konzentrat aus einem externen Behälter ist und der Aufnahmeköcher (14) vorgesehen und ausgebildet ist, den Ansaugstab (14) zu lagern und zu spülen.
